# EUROPÄISCHE PATENTSCHRIFT

(11) **EP 1 283 719 B1**
(45) Veröffentlichungstag und Bekanntmachung des Hinweises auf die Patenterteilung: **05.09.2007**
(21) Anmeldenummer: 01936257.3
(22) Anmeldetag: 20.04.2001
(51) Int. Cl.: A61K 39/35, A61K 47/48, A61P 37/00, A61P 37/08, C12N 15/63, C12N 5/10, G01N 33/53

(54) **APOPTOTIKA**
APOPTOTIC AGENTS
AGENT APOPTOTIQUE

(30) Priorität: 22.04.2000 DE 10020095
(43) Veröffentlichungstag der Anmeldung: 19.02.2003
(73) Patentinhaber: PharmedArtis GmbH, 52074 Aachen (DE)
(72) Erfinder: Barth, Stefan, 52159 Roetgen (DE); Engert, Andreas, 50931 Köln (DE); Stöcker, Michael, 52074 Aachen (DE)
(74) Vertreter: Meyers, Hans-Wilhelm
(86) Internationale Anmeldenummer: PCT/EP2001/004514
(87) Internationale Veröffentlichungsnummer: WO 2001/080880

(56) Entgegenhaltungen:
- EP-A- 0 707 065
- WO-A-97/42329
- WO-A-97/46880
- WO-A-98/29540
- WO-A-99/24565
- CA-A- 2 066 801
- US-A- 5 817 308
- ARRUDA L KARLA ET AL: "Cloning of cockroach allergen, Bla g 4, identifies ligand binding proteins (or calycins) as a cause of IgE antibody responses." JOURNAL OF BIOLOGICAL CHEMISTRY, Bd. 270, Nr. 52, 1995, Seiten 31196-31201, XP002155639 ISSN: 0021-9258
- KLIMKA A ET AL: "AN ANTI-CD30 SINGLE-CHAIN FV SELECTED BY PHASE DISPLAY AND FUSED TO PSEUDOMONAS EXOTOXIN A (KI-4(SCFV)-ETA') IS A POTENT IMMUNOTOXIN AGAINST A HODGKIN-DERIVED CELL LINE" BRITISH JOURNAL OF CANCER, LONDON, GB, Bd. 80, Juni 1999 (1999-06), Seiten 1214-1222, XP001030739 ISSN: 0007-0920
- PSARRAS KYRIAKOS ET AL: "Human pancreatic RNase1-human epidermal growth factor fusion: An entirely human 'immunotoxin analog' with cytotoxic properties against squamous cell carcinomas." PROTEIN ENGINEERING, Bd. 11, Nr. 12, Dezember 1998 (1998-12), Seiten 1285-1292, XP002180642 ISSN: 0269-2139
- ZHIVOTOVSKY BORIS ET AL: "Involvement of cellular proteolytic machinery in apoptosis." BIOCHEMICAL AND BIOPHYSICAL RESEARCH COMMUNICATIONS, Bd. 230, Nr. 3, 1997, Seiten 481-488, XP002180643 ISSN: 0006-291X
- TSE ERIC ET AL: "Intracellular antibody-caspase-mediated cell killing: An approach for application in cancer therapy." PROCEEDINGS OF THE NATIONAL ACADEMY OF SCIENCES OF THE UNITED STATES, Bd. 97, Nr. 22, 24. Oktober 2000 (2000-10-24), Seiten 12266-12271, XP002180644 October 24, 2000 ISSN: 0027-8424
- HUHN MICHAEL ET AL: "CD30-SPECIFIC IN VITRO ACTIVITY OF A NEW RECOMBINANT AND COMPLETELY HUMAN IMMUNOTOXIN." ONKOLOGIE, Bd. 23, Nr. SONDERHEFT 7, Oktober 2000 (2000-10), Seite 145 XP001030742 ISSN: 0378-584X
- DATABASE BIOSIS [Online] BIOSCIENCES INFORMATION SERVICE, PHILADELPHIA, PA, US; 2001 CHOI I ET AL: "New approaches in tumor therapy: Induction of specific apoptosis using a combined application of GrB and bispecific immunoconjugates." Database accession no. PREV200100300099 XP002180645 & JOURNAL OF CANCER RESEARCH AND CLINICAL ONCOLOGY, Bd. 127, Nr. Supplement 1, 2001, Seite S45 Eleventh Congress of the Division of Experimental Cancer Research of the German Cancer Society;Heidelberg, Germany; April 04-06, 2001 ISSN: 0171-5216
- N. TOYAMA-SORIMACHI ET AL.: "A novel ligand for CD44 is serglycin, a hematopoietic cell lineage-specific proteoglycan" THE JOURNAL OF BIOLOGICAL CHEMISTRY, Bd. 270, Nr. 13, 31. März 1995 (1995-03-31), Seiten 7437-7444,
- N. TOYAMA-SORIMACHI ET AL.: "Widespread expression of chondroitin sulfate-type serglycins with CD44 binding ability in hematopoietic cells" THE JOURNAL OF BIOLOGICAL CHEMISTRY, Bd. 272, Nr. 42, 17. Oktober 1997 (1997-10-17), Seiten 26714-26719,
- J.P. GALVIN ET AL.: "Apoptosis induced by granzyme B-glycosaminoglycan complexes: implications for granule-mediated apoptosis in vivo" THE JOURNAL OF IMMUNOLOGY, Bd. 162, 1999, Seiten 5345-5350,

## Beschreibung

Gegenstand der vorliegenden Erfindung ist ein rekombinantes Fusionsprotein, welches mindestens ein Granzym und eine zellspezifische Bindungsdomäne insbesondere humanen Ursprunges umfasst, sowie Nukleinsäuren und Vektoren, die für einen solches Fusionsprotein kodieren. Des weiteren Verfahren zur Beeinflussung des Zellwachstums und der Physiologie von Zellen mit dem erfindungsgemäßen Komplex, oder Vektoren, welche die hierfür kodierende Nukleinsäure enthalten. Die Erfindung betrifft ferner Vektoren und Wirte, um den erfindungsgemäßen Komplex zu produzieren. Ferner die Herstellung und den Vertrieb von Arzneimitteln, auf Basis des erfindungsgemäßen Komplexes oder dafür kodierender Vektoren, zur Behandlung von Erkrankungen, die auf einer pathologischen Vermehrung und/oder einer erhöhten Aktivität von strukturell definierten Zellpopulationen beruhen. Dies gilt insbesondere für Tumorerkrankungen, Allergien, Autoimmunerkrankungen, chronische Entzündungsreaktionen oder Gewebeabstoßungsreaktionen.

Bei der medikamentösen Behandlung von Tumoren, Autoimmunerkrankungen, Allergien und Gewebeabstoßungsreaktionen tritt nachteilig in Erscheinung, dass die heute verfügbaren Medikamente wie Chemotherapeutika, Kortikosteroide und Immunsuppressiva aufgrund ihrer relativen Unspezifität ein mitunter erhebliches Potential an Nebenwirkungen aufweisen. Man hat versucht, dies durch verschiedene therapeutische Konzepte abzumildern. Insbesondere die Verwendung von Immuntherapeutika ist ein Ansatz, der zur Erhöhung der Spezifität von Medikamenten, insbesondere bei der Tumorbehandlung, geführt hat.

Ist das Immuntherapeutikum ein Immuntoxin, wird ein monoklonaler Antikörper (moAk) oder ein Antikörperfragment, welches eine kinetische Affinität zu Oberflächenmarkern von Tumorzellen aufweist, mit einem zytotoxischen Reagenz gekoppelt. Ist das Immuntherapeutikum ein Antiimmunkonjugat zur Behandlung von Autoimmunerkrankungen, Gewebeabstoßungsreaktionen oder Allergien, wird eine für die Pathogenese relevante Struktur oder ein Fragment derselben an eine Toxinkomponente gekoppelt. Es hat sich herausgestellt, dass sich Immuntoxine bei klinischem Einsatz durch hohe Immunogenität auszeichnen können. Dadurch kommt es im Patienten zur Ausbildung von neutralisierenden Antikörpern, die das Immuntoxin inaktivieren. Im allgemeinen ist eine mehrmalige und/oder kontinuierliche Gabe der Therapeutika für langfristige kurative Effekte unumgänglich. Dies wird besonders deutlich bei der Supprimierung von Gewebeabstoßungsreaktionen nach Transplantationen, oder bei der Behandlung von Autoimmunerkrankungen, durch die teilweise belegte genetisch bedingte Prädominanz zu einer pathogenen Autoimmunreaktion.

### Rekombinante Fusionsproteine auf der Basis autologer, apoptoseinduzierender Proteasen (Apoptotika)

Um einen direkten therapeutischen Effekt an den Zielzellen zu erzielen, hat man Antikörper mit radioaktiven Elementen oder mit Toxinen zu sogenannten Radioimmunkonjugaten oder Immuntoxinen (IT) verknüpft. Beim Einsatz von radioaktiv-markierten anti-B-Zell-moAk bei B-Zelllymphomen konnten Tumorregressionen und sogar vollständige Remission beobachtet werden (Jurcic, J. G. and Scheinberg, D. A. 1995; Kaminski, M. S. *et al.* 1996; Press, O. W. *et al.* 1993). Die Ergebnisse mit moAk gegen solide Tumoren hingegen waren eher ernüchternd (LoBuglio, A. F. and Saleh, M. N. 1992; Saleh, M. N. *et al.* 1992). Eine Erklärung hierfür scheint die zu geringe Tumorpenetration aufgrund ihrer Größe, insbesondere bei schlecht vaskularisierten Tumoren, zu sein. In der weiteren Entwicklung wurden daher Antikörperfragmente oder zielzellspezifische Liganden an die entsprechenden Effektoren gekoppelt. Rationale für die Miniaturisierung waren eine bessere Gewebe- und Tumorpenetration durch verbesserte Diffusionseigenschaften, sowie eine erhoffte geringere Immunogenität durch Reduzierung der antigenen Determinanten (Pirker, R. 1988; Yokota, T. *et al.* 1992). Verbesserte Klonierungstechniken ermöglichen die vollständig rekombinante Herstellung von IT. Die genetischen Informationen der variablen Domänen eines moAk werden dabei über einen synthetischen (Gly₄Ser)₃-Linker miteinander zur einem einzelsträngigen Fragment verknüpft (scFv). Über eine weitere Fusion auf DNA-Ebene, wird die katalytische Domäne, etwa eines Toxins, an das scFv fusioniert (Chaudhary, V. *et al.* 1990; Chaudhary, V. *et al.* 1989). Neben der Verwendung von scFv können auch Liganden für Tumorzell-spezifische Rezeptoren an die Toxine gekoppelt werden (Klimka, A. *et al.* 1996). Neben diesen aktiven Bindern können auch passive Bindungsstrukturen für das zellspezifische Targeting eingesetzt werden. Der wesentliche Unterschied beruht auf der Tatsache, dass Immunglobuline wie Antikörper und T-Zell-Rezeptoren Autoantigene und Allergene "erkennen". Ist das Immuntherapeutikum also ein Antiimmunkonjugat zur Behandlung von Autoimmunerkrankungen, Gewebeabstoßungsreaktionen oder Allergien, wird eine für die Pathogenese relevante Struktur oder ein Fragment derselben an eine Toxinkomponente gekoppelt (Brenner, T. *et al.* 1999).

Die bislang am meisten verwendeten und damit am besten charakterisierten peptidischen Zellgifte sind die bakteriellen Toxine Diphtherietoxin (DT) (Beaumelle, B. *et al.* 1992; Chaudhary, V. *et al.* 1990; Kuzel, T. M. *et al.* 1993; LeMaistre, C. *et al.* 1998), *Pseudomonas* Exotoxin A (PE) (Fitz Gerald, D. J. *et al.* 1988; Pai, L. H. and Pastan, I. 1998), sowie das aus Pflanzen stammende Ricin-A (Engert, A. *et al.* 1997; Matthey, B. *et al.* 2000; O'Hare, M. *et al.* 1990; Schnell, R. *et al,* 2000; Thorpe, P. E. *et al.* 1988; Youle, R. J. and Neville, D. M. J. 1980). Der zytotoxische Wirkmechanismus ist bei all diesen Toxinen trotz ihres unterschiedlichen evolutionären Hintergrundes gleich. Die katalytische Domäne inhibiert die Proteinbiosynthese durch Modifizierung des für die Translation wichtigen Elongationsfaktors EF-2 oder der Ribosomen direkt, so dass EF-2 nicht mehr binden kann (Endo, Y. *et al.* 1987; Iglewski, B. H. and Kabat, D. 1975).

Die systemische Applikation von Immuntoxinen führt bei den meisten der bisher verwendeten Konstrukte zu mehr oder weniger starken Nebenwirkungen. Neben dem 'Vaskular-Leak' Syndrom, (Baluna, R. and Vitetta, E. S. 1997; Schnell, R. *et al.* 1998; Vitetta, E. S. 2000) kommt es in Abhängigkeit von dem verwendeten Konstrukt und der applizierten Dosis zu Thrombozytopenie, Hämolyse, Niereninsuffizienz und Übelkeit. Es konnten auch dosisabhängige und reversible Leberschädigungen beobachtet werden (Battelli, M. G. *et al.* 1996; Grossbard, M. L. *et al.* 1993; Harkonen, S. *et al.* 1987). Die beim Einsatz der Immunkonjugate oder Immuntoxine zu beobachtende Immunogenität der verwendeten Konstrukte ist neben den dokumentierten Nebenwirkungen das zentrale Problem der Immuntherapie (Khazaeli, M. B. *et al.* 1994). Dies bezieht sich insbesondere auf die humorale Abwehr gegen die verwendeten katalytischen Domänen wie Ricin (HARA) (Grossbard, M. L. *et al.* 1998), PE (Kreitman, R. J. *et al.* 2000), oder DT (LeMaistre, C. F. *et al.* 1992). Theoretisch können alle nicht-humanen Strukturen eine Immunantwort provozieren. Einer wiederholten Gabe von Immuntoxinen und Immunkonjugaten sind damit Grenzen gesetzt. Logische Konsequenz dieser Problematik ist die Entwicklung humaner Immuntoxine (Rybak, S. *et al*. 1992).

Humane Toxine für den Einsatz in Immuntoxinen rekrutieren sich bisher ausschließlich aus sogenannten Ribonukleasen. Nachdem das zytotoxische Potential der humanen RNase A durch Mikroinjektion in Zellen gezeigt werden konnte (Rybak, S. *et al.* 1991), wurde diese chemisch an einen anti-CD5 moAK gekoppelt und erfolgreich in einem *in vivo*-Modell getestet (Newton, D. L. *et al.* 1992). Da humane RNasen in extrazellulären Flüssigkeiten, Plasma und Geweben vorhanden sind, geht man davon aus, dass diese bei einem Einsatz in Immuntoxinen weniger immunogen sind. Angiogenin (ANG), ein 14 kDa Protein mit einer 64%igen Sequenzhomologie zu RNase A, wurde erstmals aus tumorzellkonditioniertem Medium isoliert, wo es aufgrund seiner Fähigkeit Gefäßwachstum zu induzieren entdeckt wurde (Fett, J. W. *et al.* 1985). Es konnte gezeigt werden, dass die t-RNA spezifische RNase-Aktivität von Angiogenin zytotoxisches Potential hat (Saxena, S. K. *et al.* 1992; Shapiro, R. *et al.* 1986). Entsprechend chemisch konjugierte Immuntoxine zeigten in der Folge eine zellspezifische toxische Aktivität (Newton, D. *et al.* 1996; Yoon, J. M. *et al.* 1999). Um die Wirksamkeit von ANG-basierten scFv-Immuntoxinen zu evaluieren, wurden verschiedene Konformationen von ANG mit dem epidermalen Wachstumsfaktor (EGF) konstruiert und *in vitro* erfolgreich getestet (Yoon, J. M. *et al.* 1999). Ein weiteres Mitglied der RNase-Superfamilie ist das eosinophile Neurotoxin (EDN). Für das 18,4 kDa große EDN konnte bisher nur die direkte Neurotoxizität beschrieben werden. Auf Grundlage der dokumentierten Potenz, wurden verschiedene EDN-basierte Immuntoxine konstruiert und *in vitro* ebenfalls erfolgreich getestet (Newton, D. *et al.* 1994; Zewe, M. *et al.* 1997).
Neuere Untersuchungen zeigen, dass ANG durch einen zelleigenen zytoplasmatischen Ribonukleaseinhibitor (RI) blockiert werden kann. Dies schränkt die Wirksamkeit von auf ANG basierenden IT bei RI(+)-Zielzellen ein (Leland, P. A. *et al.* 1998).

Der Erfindung liegen die folgenden technischen Probleme zugrunde:
Herabsetzung der Immunogenität der Immuntherapeutika, Herabsetzung der reduzierten Wirkungsaktivität durch unspezifische Inaktivierung sowie Verbesserung der durch wirtseigene spezifische Inhibitoren reduzierten Wirkungsaktivität.

Gelöst werden diese Probleme durch ein rekombinantes Fusionsprotein, das gebildet ist aus mindestens einer Komponente A und mindestens einer Komponente B, wobei die Komponente A eine Bindungsaktivität für zelluläre Oberflächenstrukturen und Komponente B ein Granzym ist.

Das erfindungsgemäße Fusionsprotein umfasst mindestens zwei Domänen, eine Effektordomäne und eine Bindungsdomäne.

Die Effektordomäne besteht aus einem Granzym vorzugsweise Granzyme B im Menschen. Die Bindungsdomäne besteht aus einer Struktur, welche eine Bindung und Internalisierung in strukturell definierte Zielzellen ermöglicht.

Von Vorteil ist, dass es sich bei der katalytischen Domäne um ein körpereigenes Protein oder ein Derivat desselben handelt und dadurch bedingt die zu erwartende Immunogenität drastisch reduziert ist. Insbesondere bei den im Zusammenhang mit Autoimmunerkrankungen, Allergien und Gewebeabstoßungsreaktionen zu eliminierenden reaktiven Zellen des Immunsystems, handelt es sich im physiologischen Sinne um normale Zellen. Diese Zellen lassen eine normale Empfindlichkeit gegenüber natürlichen apoptoseinduzierenden Elementen erwarten.

Vorzugsweise weist das erfindungsgemäße Fusionsprotein neben den Komponenten A und B eine oder mehrere supplementäre Komponenten S auf. Der Fachmann weiß aufgrund seiner Erfahrung, dass zusätzliche Merkmale und Eigenschaften entscheidenden Beitrag für die effiziente Herstellung und/oder Wirksamkeit der erfindungsgemäßen Fusionsproteine haben können. Bedingt durch die Verschiedenheit der mit den erfindungsgemäßen Fusionsproteinen zu behandelnden Erkrankungen kann eine Adaption der Fusionsproteine an die jeweiligen besonderen Gegebenheiten notwendig sein.

Vorzugsweise wird die Komponente A des erfindungsgemäßen Fusionsproteins ausgewählt aus der Gruppe aktiv bindender Strukturen, bestehend aus Antikörpern, deren Derivate und/oder Fragmente, synthetischen Peptiden oder chemischen Molekülen, Liganden, Lektinen, Rezeptorbindungsmolekülen, Cytokinen, Lymphokinen, Chemokinen, Adhäsionsmolekülen, die an Cluster of Differentiation (CD-Antigene), Zytokin-, Hormon-, Wachstumsfaktor-Rezeptoren, Ionenpumpen, kanalbildende Proteine binden und deren Derivaten, Mutanten oder Kombinationen davon.

In einer weiteren Ausführungform des erfindungsgemäßen Fusionsproteins ist dieses dadurch gekennzeichnet, dass die Komponente A ausgewählt wird aus der Gruppe passiv gebundener Strukturen aus Allergenen, peptidischen Allergenen, rekombinanten Allergenen, Allergen-idiotypischen Antikörpern, autoimmunprovozierenden Strukturen, Gewebeabstoßungs-induzierenden Strukturen und deren Derivate, Mutanten oder Kombinationen davon.

Die Komponente B des erfindungsgemäßen Fusionsproteins besitzt insbesondere proteolytische Eigenschaften oder ist mindestens eine Protease, deren Derivate, Mutante oder Kombination davon.

Keine der bisher in Immuntoxinen beschriebenen Effektordomänen nutzen proteolytische Eigenschaften und initiieren direkt die natürlichen Mechanismen zur Einleitung der Apoptose in den Zielzellen. Die Effekte bislang beschriebener Immuntoxine beruhen immer auf einer Störung oder Inhibition der Translation in den Zielzellen. Die daraus resultierende Beeinträchtigung der Vitalität der Zellen kann indirekt zu einer Einleitung der Apoptose führen (Bolognesi, A. *et al.* 1996; Keppler-Hafkemeyer, A. *et al.* 1998; Keppler-Hafkemeyer, A. *et al.* 2000). Vorzugsweise aktiviert die Komponente B des erfindungsgemäßen Fusionsproteins Komponenten der zellinhärenten Apoptose direkt und induziert damit die Apoptose in den über die Bindung von Komponente A definierten Zellen.

Besonders bevorzugt ist Granzym B (GB) oder ein Derivat davon als Komponente B des erfindungsgemäßen Fusionsproteins. Die serinabhängige und aspartamspezifische Protease Granzym B ist von besonderem Interesse. Granzym B ist eine Komponente der zellulären Immunabwehr, die nach Aktivierung zytotoxischer T-Zellen (CTL) oder natürlicher Killerzellen (NK) aus den zytotoxischen Granula dieser Zellen ausgeschüttet wird (Kam, C. M. *et al.* 2000; Shresta, S. *et al.* 1998). Nach perforinabhängiger Translokation von Granzym B in das Zytoplasma attackierter Zellen wird eine proteolytische Kaskade initiiert, die in der Apoptose der Zielzelle endet (Greenberg, A. H. 1996). Die genaue Funktion des mit dem Granzym B ausgeschütteten Perforin wird zur Zeit noch diskutiert, ist aber alleine nicht in der Lage, Apoptose zu induzieren. Perforin lagert sich in der Zellmembran zu 12-18meren zusammen und bildet dadurch 15-18 nm Poren. Ursprünglich ging man davon aus, dass Granzym B durch diese Poren in das Zytoplasma der Zielzellen gelangt. Das 32 kDa Protein Granzym B ist für diese Passage aber zu groß. Wahrscheinlicher ist die Annahme, das nach Bindung des Granzym B an Perforin und sukzessiver Internalisierung dieses Komplexes, Perforin die endosomalen Freisetzung des Granzym B unterstützt (Jans, D. A. *et al.* 1996). In den letzten Jahren konnten verschiedene Proteine identifiziert werden, welche durch GB-vermittelte Spaltung aktiviert und in direktem Zusammenhang mit der Apoptose stehen. So konnte *in vitro* die GB-bedingte proteolytische Aktivierung verschiedener Procaspasen, insbesondere 3 und 8, dokumentiert werden (Fernandes-Alnemri, T. *et al.* 1996; Srinivasula, S. M. et *al.* 1996), welche zu den zentralen Proteasen bei der Apoptose gerechnet werden (Nicholson, D. W. and Thornberry, N. A. 1997). Weitere zytotoxische Aktivitäten entfaltet Granzym B im Zellkern. Granzym B wird, nachdem es in das Zytoplasma der Zielzelle eingedrungen ist, relativ schnell caspaseabhängig in den Zellkern transloziert (Pinkoski, M. J. *et al.* 2000). Dort ist Granzym B zum Beispiel in der Lage, das nukleare Matrixantigen und die poly-(ADP-Ribose)-Polymerase zu schneiden (Andrade, F. *et al.* 1998). Eine schnelle Apoptose konnte in Zellen beobachtet werden, nachdem Granzym B im Zellkern akkumulierte (Trapani, J. A. *et al.* 1998; Trapani, J. A. *et al.* 1998). Neuere Daten belegen die Initiierung der Apoptose über die direkte proteolytische Spaltung von Bid; einem Mitglied der Bcl-2-Familie mit nur einer BH3-Domäne. Die nach der Spaltung verkürzte Form tBid lagert sich in die mitochondriale Membran ein und depolarisiert diese. Dadurch wird die Freisetzung von Cytochrom C und einem apoptoseinduzierenden Faktor aus den Mitochondrien in das Zytoplasma induziert, was den Zelltod entscheidend beschleunigt (Sutton, V. R. *et al.* 2000). Weitere caspaseunabhängige toxische Eigenschaften des Granzym B konnten beschrieben werden, wobei der zu Grunde liegende Mechanismus bisher ungeklärt ist (Beresford, P. J. *et al.* 1999; Sarin, A. *et al.* 1997).

Weitere Ausführungsformen der erfindungsgemäßen Fusionsproteins können eine oder mehrere verschiedene Komponenten S enthalten. Der Fachmann ist aufgrund seines Wissens in der Lage, die Vorteile und Notwendigkeit von zusätzlichen Komponenten und/oder Merkmalen in Zusammenhang mit den erfindungsgemäßen Fusionsproteinen zu bewerten. Die Komponenten S können zum Beispiel folgenden Zwecken dienen:
- Der induzierbaren Regulierung der Syntheseleistung (z. B. induzierbare Promotoren).
- Der Steuerung der Proteinbiosynthese (z. B. Leader-Sequenz).
- Der Aufreinigung des Komplexes oder seiner Komponenten (z. B. His-Tag, Affnitats-Tags).
- Der Translokation des Apoptotika in die Zielzellen (z. B. Translokationsdomäne, amphiphatische Sequenzen).
- Der intrazellulären Aktivierung der Komponente B (synthetisches Pro-Granzyme B, amphiphatische Sequenzen).

Gegenstand der Erfindung sind auch Nukleinsäuremoleküle. oder Vektoren, die für das erfindungsgemäße Fusionsprotein oder für einzelne Komponenten zur Herstellung des Fusionsproteins kodieren. Den Erfindern ist es gelungen, die Expression der Apoptotika in eukaryontischen Zellen humanen Ursprungs zu dokumentieren. Dies legt die Eignung von Nukleinsäuren, die für ein erfindungsgemäßes Fusionsprotein kodieren, auch für gentherapeutische Ansätze nahe. Der Fachmann ist aufgrund seines Wissens in der Lage, die vielfältigen Aspekte und Möglichkeiten gentherapeutischer Interventionen in Zusammenhang mit den verschiedenen zu behandelnden Erkrankungen zu erkennen. Neben der lokalen Applikation relativ unspezifischer Vektoren (*eg.* kationische Lipide, nonvirale, adenovirale und retrovirale Vektoren) wird in näherer Zukunft auch eine systemische Applikation mit modifizierten zielzellspezifischen Vektoren möglich werden. Bis zur Verfügbarkeit solcher Systeme bietet die gezielte Transfektion definierter Zellpopulationen *ex vivo* und deren Rückführung in den zu behandelnden Organismus eine interessante Alternative (Chen, S. *et al.* 1997).

Auch Organismen, die nach Transformation oder Transfektion mit den erfindungsgemäßen Nukleinsäuremolekülen oder Vektoren vollständige erfindungsgemäße Fusionsproteine synthetisieren, werden erfindungsgemäß beansprucht.

Arzneimittel enthaltend ein erfindungsgemäßes Fusionsprotein. Typischerweise werden die erfindungsgemäßen Fusionsproteine in physiologisch verträglichen Darreichungsformen gegeben. Dazu gehören z.B. Tris-, NaCl-, Phosphatpuffer und alle zugelassenen Puffersysteme, insbesondere auch Puffersysteme die sich durch Zusatz zugelassener Proteinstabilisatoren auszeichnen. Die Applikation erfolgt insbesondere über parenterale, intravenöse, subkutane, intramuskuläre, intratumorale, transnasale Applikation sowie Applikation über Schleimhäute.

Die Dosis der zu verabreichenden erfindungsgemäßen Fusionsproteins muss für jede Applikation bei jeder neu zu therapierenden Erkrankung durch klinische Phase I-Studien (Dosis-Eskalationsstudien) ermittelt werden.

Nukleinsäuren oder Vektoren, die für ein erfindungsgemäßes Fusionsprotein kodieren, werden vorteilhafterweise in physiologisch verträglichen Darreichungsformen gegeben. Dazu gehören z.B. Tris-, NaCl-, Phosphatpuffer und alle zugelassenen Puffersysteme, insbesondere auch Puffersysteme die sich durch Zusatz zugelassener Stabilisatoren für die zu verwendenden Nukleinsäuren und/oder Vektoren auszeichnen. Die Applikation erfolgt insbesondere über parenterale intravenöse, subkutane, intramuskuläre, intratumorale, transnasale Applikation sowie Applikation über Schleimhäute.

Das erfindungsgemäße Fusionsprotein und/oder, dafür kodierende Nukleinsäuremoleküle können zur Herstellung eines Arzneimittels zur Behandlung maligner Erkrankungen, Allergien, Autoimmunreaktionen, chronischen Entzündungsreaktionen oder Gewebeabstossungsreaktionen verwendet werden.

Am Beispiel des anti-CD30 Apoptotikums Ki-4(scFv)-Granzym B (siehe unten) (KGbMH) konnte die zytotoxische Wirksamkeit eines auf der vorliegenden Erfindung beruhenden Fusionsproteins am Beispiel der Hodgkinzelllinie L540Cy bewiesen werden. Die Sezernierung dieses funktionellen Fusionsproteins aus eukaryontischen Zellen demonstriert zudem die potenzielle Eignung der erfindungsgemäßen Proteine für eine gentherapeutische Anwendung.

### Herstellung des rekombinanten CD30-spezifischen Apoptotikums Ki-4(scFv)-Granzym B (KGbMH)

### Methoden

### Bakterien, Oligonukleotide und Plasmide

*E.coli* XL1-blue wurden für die Propagierung der Plasmide verwendet. Synthetische Oligonukleotide wurden von der Firma MWG (Martinsried, Deutschland) bezogen. Die Präparation der Plasmide erfolgte nach der Alkalischen-Lyse-Methode und wurden mit Hilfe der Plasmid-Purification-Kit's von Qiagen (Hilden, Deutschland) aufgereinigt.

### Zelllinien

Alle verwendeten Zelllinien (Tabelle 1) wurden in Komplexmedium (RPMI-1640, 10% FCS,50 µg/ml Penicillin und 100 µg/ml Streptomycin) bei 37°C in einer Atmosphäre von 5% CO₂ kultiviert.
Die Anreicherung/Kultivierung transfizierter Zellen erfolgte unter Selektionsdruck mit 100 µg/ml Zeocin.

**Tabelle 1 : Verwendete Zelllinien**

| Zelllinie | Ursprung | Referenz |
|---|---|---|
| 293T | Humane, embryonale Nierenzellen | (Graham, F. L. *et al.* 1977) |
| L540Cy | Hodgkin-Lymphom | (Kapp, U. *et al.* 1992) |
| IMR5 | Neuroblastom | (Bukovsky, J. *et al.* 1985) |

### Klonierungstechniken

### Rekombinante Techniken

Für die Klonierung, Analyse und Konstruktion der verschiedenen DNA-Fragmente und die verwendeten Plasmide wurden Standardtechniken angewendet (Sambrook, J. *et al.* 1989). Die jeweiligen Gebrauchsvorschriften der Hersteller für die Verwendung ihrer Produkte, insbesondere bei Enzymen und Kits, wurden entsprechend berücksichtigt. Verwendet wurden Enzyme und

Kits der Firmen Qiagen, Roche, NEB, AGS und Genecraft.

### cDNA Herstellung

Humane RNA wurde aus Vollblut mit Hilfe des QIAamp RNA Blood Mini Kits gewonnen. Die so gewonnene RNA wurde mit dem First-Strand cDNA Synthesis Kit von Pharmacia Biotech in cDNA umgeschrieben. Neben den im Kit bereitgestellten Primern wurden für die Erststrangsynthese auch die spezifischen Primer für Granzym B eingesetzt.

### PCR

Die Erststrang-cDNA wurde sofort in einer PCR mit den GB-spezifischen Primern amplifizieren. Das Design der Primer orientierte sich an den in der PubMed-Gendatenbank unter Accession-Nummer NM_004131 verfügbaren Sequenzdaten.

Die PCR wurde unter Standardbedingungen in einem Primus-Thermocycler (MWG, Martinsried) durchgeführt. Standard Programmierung: 96°C,5'; 30x (96°C,1'; 60°C,1'; 72°C, 1'); 72°C, 4'.

### Klonierung der eukaryontischen Expressionsplasmide

Grundplasmid für die Klonierung und eukaryontische Expression der rekombinanten GB-Fusionsproteine war das pSecTag2 (Invitrogen, Niederlande). Nach verschiedenen Umklonierungen und Modifikationen im Bereich der MCS und der Markerepitope, waren wir in der Lage, das Ki-4(scFv) aus dem bakteriellen Expressionsvektor pBM1.1-Ki-4 (Barth, S. *et al.* 2000) und über Xho I/Cel II das Granzym B in die neugestalteten pMS-Plasmide zu klonieren.

Weitere hiervon abgeleitete pMS-Plasmide enthielten die IVS und die IRES-Sequenz sowie die hieran anschließende Sequenz für das Reportergen EGFP (green fluorescent protein) aus dem pIRES-EGFP-Plasmid (Clontech, USA). Dies ermöglichte eine unkomplizierte Bestimmung der Transfektionsrate und vereinfachte die Selektion transfizierter Zellpopulationen.

Alle verwendeten Plasmide verfügten somit über folgende Merkmale:
- CMV-Promotor für die konstitutive Expression der GB-Konstrukte
- Muriner Ig-Kappa-Leader für die Sezernierung der Immuntoxine
- BGH-Polyadenylierungssequenz
- Zeocin-Resistenzgen für die Selektion in Eukaryonten
- ColE1-Ori für die Replikation in Prokaryonten
- Ampicillinresistenzgen für die Selektion in Prokaryonten

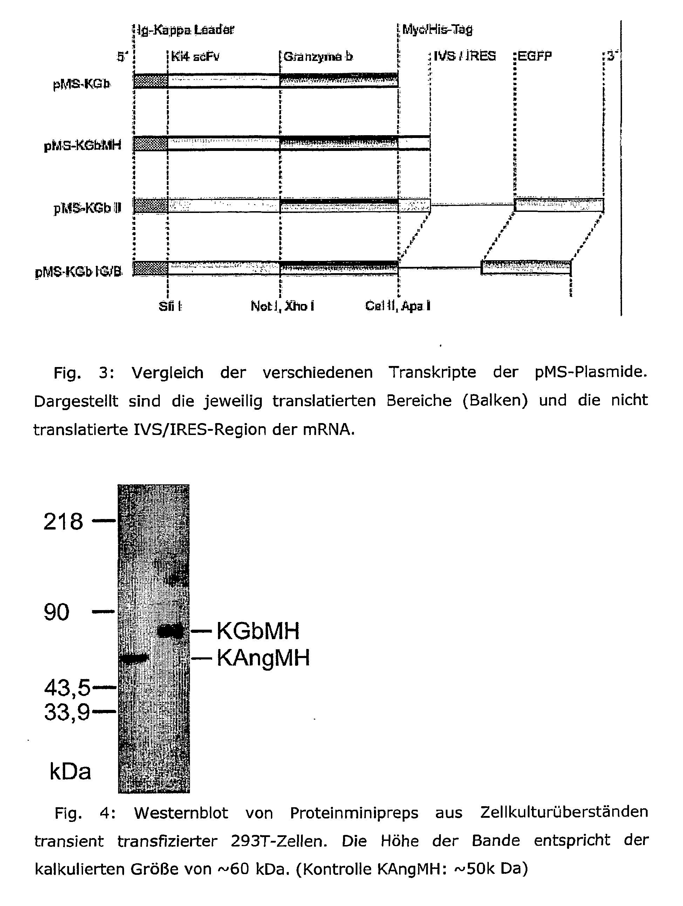

Die Unterschiede der verwendeten Plasmide sind in Fig. 3 dargestellt. Das Plasmid pMS-KGb kodiert im Gegensatz zum pMS-KGbMH für ein Ki-4(scFv)-Granzym B-Fusionsprotein ohne Myc- und His-Tag und sollte klären, ob C-Terminal angefügte Sequenzen die Funktionalität des Immuntoxins beeinflussen. Das Anfügen der IVS/IRES/EGFP-Sequenz an diese beiden Konstrukte sollte einen möglichen Effekt des EGFP auf die Immuntoxin-Syntheseleistung der Zellen klären (*eg*. pMS-KGb IG/B und pMS KGb II).

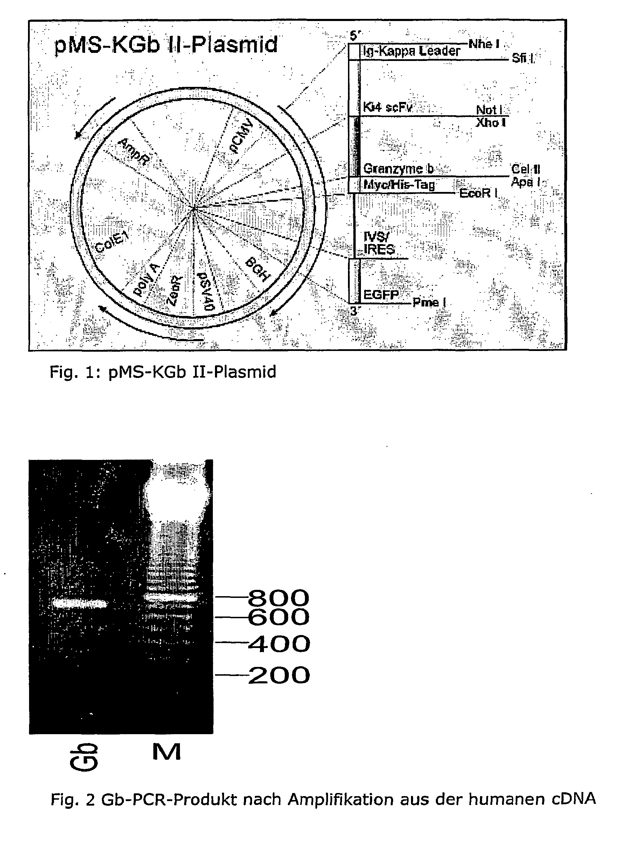

Ein Beispiel für die vollständige Struktur der pMS-Plasmide ist in Fig. 1 dargestellt.

### Sequenzierung

Die DNA-Sequenzierung wurde nach der Didesoxy-Kettenabbruch-Methode (Sanger, F. *et al.* 1977) durchgeführt. Das verwendete *ABI PRISM BigDye Terminator Cycle Sequencing Ready Reaction Kit* enthält alle notwendigen Komponenten für die Reaktion außer Template und Primer. Die Sequenzreaktionen wurden an einem Primus-96plus Thermocycler mit Heizdeckel (MWG-Biotech) ohne PCR-Öl durchgeführt.

### Transfektion eukaryontischer Zellen

Die Transfektion eukaryontischer Zellen wurde mit TransFast®, einem synthetischen kationischen Lipid (Promega), durchgeführt. Das verwendete Plasmid pMS-KGb II verfügte über das EGFP- Reportergen. Die Transfektionsraten bei 293T-Zellen lag zwischen 50-80%, was durch Auszählen der grün fluoreszierenden Zellen am Fluoreszenzmikroskop bestimmt werden konnte. Die Transfektion wurde nach dem Herstellerprotokoll durchgeführt. Nach 3 Tagen wurden die transfizierten Zellen in kleine Zellkulturflaschen umgesetzt und unter Zeocin®-Selektionsdruck (100 µg/ml) weiter kultivieren und selektioniert.

### Aufreinigung der rekombinanten Proteine

Die Proteinaufreinigungen wurden ausschließlich mit NiNTA (Qiagen) durchgeführt. Diese Methode der Immobilisierten-Metall-AffinitätsChromatographie (IMAC) nutzt die ladungsbedingte Affinität von Histidin-Clustern (HisTag) in Proteinen, um an über NTA immobilisierte Ni²⁺ -Ionen zu binden (Hochuli, V. 1989; Porath, J. *et al.*1975)*.* Imidazol, ein Histidinanalog, kompetiert bei der Elution der rekombinanten Proteine mit dem His-Tag.

### Proteinminiprep

Die Proteinminipräparationen erfolgten in Anlehnung an die Qiagenprotokolle (*The Expressionist* 07/97) zur nativen Aufreinigung von Proteinen mit einem His-Tag (Crowe, J. *et al.* 1994). Das NiNTA wurde vor Verwendung 3x mit 1fach Inkubationspuffer gewaschen und darin bei 4°C gelagert (NiNTA 50%ig). Durchführung des Protokolls bei RT, alle Zentrifugationsschritte bei 6000 U/min in einer Tischzentrifuge.
1,2-1,5 ml Zellkulturüberstand 2' zentrifugieren um Zellen und Zellbestandteile zu sedimentieren. 900 µl dieses Zellkulturüberstandes mit 300 µl 4fach Inkubationspuffer (200 mM NaH₂PO₄, pH 8,0; 1,2 M NaCl; 40 mM Imidazol) und 30 µl 50%iges NiNTA in einem 1,5 ml-Eppendorfgefäß versetzen. 1 h unter schütteln inkubieren. 1' zentrifugieren und den Überstand verwerfen. Das NiNTA-Pellet 2x in 175 µl 1fach-Inkubationspuffer resuspendieren und den Überstand nach 1' Zentrifugation jeweils verwerfen. 30 µl Elutionspuffer (50 mM NaH₂PO₄, pH 8,0; 300 mM NaCl; 250 mM Imidazol) zum NiNTA-Pellet zugeben und 20 min bei RT unter schütteln inkubieren. Das Pellet 1' abentrifugieren und den Überstand mit dem aufgereinigten Protein in ein neues Eppendorfreaktionsgefäß überführen.

### Aufreinigung von Proteinen über NiNTA Affinitätssäulen

Die Proteinaufreinigung über NiNTA-Affnitätssälen wurde an einer Bio-Rad Biologic Workstation mit einem Fraktionssammler Modell 2128 und einem entsprechenden Controller-PC durchgeführt. Die verwendeten Puffer sind identisch zu den im Proteinminiprep verwendeten. Nach Elution der rekombinanten Proteine wurden diese aufkonzentriert und umgepuffert.

### Proteinkonzentrierung und Umpufferung mittels Ultrafiltration

Um die aufgereinigten Proteine in verschiedenen Tests einzusetzen, mussten die von der NiNTA-Säule eluierten Proben aufkonzentriert, deren Konzentration bestimmt und umgepuffert werden. Durch das Umpuffern in PBS wurde auch das für Zellen schädliche Imidazol des Elutionspuffers aus den Präparationen entfernt.

Für die Aufkonzentrierung und Umpufferung wurde eine Amicon 2000 Ultrafiltrationskammer der Firma Amicon und Diaflo Ultrafiltrations-Membranen mit einer Porenausschlussgröße für Proteine < 10 kDa verwendet. Unter Hochdruck aus einer Stickstoffgasflasche wurden die GB-Fusionsproteine aufkonzentriert und anschließend umgepuffert.

Die konzentrierte Proteinlösung wurde nach sterilfiltrieren in einem 1,5 ml-Reaktionsgefäß bei 4°C lagern.

### Proteinmengenbestimmung

Zur Bestimmung der Gesamtproteinkonzentration der eingeengten Proteinproben wurde ein modifizierter Lowry-Assay (Lowry, O. H. *et al.* 1951) (Bio-Rad-DC-Protein-Test) eingesetzt.

Neben der Gesamtproteinbestimmung wird mit den Proben eine SDS-PAGE-Gelelektrophorese mit anschliessender Coomassie-Färbung des Gels durchgeführt. Dies erlaubte eine Abschätzung über den Anteil des aufgereinigten rekombinanten Proteins am Gesamtprotein in der Probe.

### SDS-Polyacrylamid-Gelelektrophorese (SDS-PAGE)

Für die Gelelektrophorese von Proteinen wurden ausschließlich vorgefertigte lineare 4-15% Tris-HCl-Gradientengele in der entsprechenden Ready Gel Cell Gelkammer (Bio-Rad) verwendet. Nach 10' Kochen der Proben in nicht reduzierendem 4x Roti-Load-Probenpuffer (Fa. Roth) wurden die Proben auf die Gele aufgetragen. Gelelektrophorese in einem Tris/Glycine/SDS-Laufpuffer (Bio-Rad) 0,6 h mit 200 V bei RT.

### Western-Blot

Durchführung nach dem Tankverfahren in einer Mini Trans-Blot-Cell-Kammer (Bio-Rad) auf PVDF-Hybond-Membranen (Amersham/Pharmacia). Transferbedingungen: 1,2 h bei 500 mA in Blotpuffer (25 mM Tris-Base; 192 mM Glycin, pH 8,3; 20% Methanol).

### Immunfärbung von Westernblots

Die Immunfärbung der geblotteten Proteine wurde nach Standardmethoden durchgeführt. Der Nachweis der GB-Fusionsproteine erfolgte mit dem Anti-Penta-His-Antikörper von Qiagen (1/5000 Vol. in TTBS (1,4 g/l Trisbase; 6,05 g/l Tris-HCl; 8,78 g/l NaCl, pH 7.5; 0,05% Tween20; 0,1% BSA)). Die Detektion wurde über einen ein HRP-konjugierter Esel-Anti-Maus IgG (Dianova) (1/10000 Vol. in TTBS) durchgeführt. Für die finale Chemilumeneszenzreaktion wurde das ECL-System (Amersham Pharmacia) verwendet und entsprechende X-Ray Filme (Roche) belichtet.

### Coomassie-Färbung von SDS-PAGE-Gelen

SDS-PAGE-Gele wurden in die Färbelösung (0.25% Coomassie Brilliant Blue R250; 45% Methanol; 45% ddH₂O; 10% Essigsäure) gelegt und 1 h auf einem Rotationsschüttler inkubiert. Danach wurden die SDS-PAGE-Gele mehrfach in einer Entfärbelösung (45% Methanol; 45% ddH₂O; 10% Essigsäure) gewaschen und abschließend mit H₂O gereinigt.

### In-vitro-Charakterisierung der rekombinanten Proteine

### FACS-Analysen

Die Bindungsfähigkeit der von den transfizierten Zellen sezernierten KGb-Konstrukte wurde über Zelldurchflusszytometrie bestimmt (Barth, S. *et al.* 1998). Zellsuspensionen mit 2x10⁵ Zellen wurden kurz in PBS/BSA/N₃ (PBS mit 0,2% BSA und 0,05% Natriumazid) gewaschen und anschließend mit Zellkulturüberständen oder aufgereinigtem GB-Apoptotika in PBS/BSA/N₃ 30' bei 4°C inkubiert. Nach 3 Waschungen wurden die Zellen 30'bei 4°C mit 1/1000 Vol. anti-Penta-His in PBS/BSA/Azid inkubiert. Erneutes 3maliges Waschen der Zellen und 15' Inkubation mit 1/50 Vol. FITC-Ziege-anti-Maus Ak in PBS/BSA/Azid. Nach 3 weiteren Waschungen in PBS/BSA/Azid wurde die Zellsuspension mit 2 µl 6,25 mg/ml Propidiumjodid versetzt und sofort in einem FACS-Calibur (Becton Dickinson, Heidelberg, Germany) analysiert.

### XTT-Viabilitätstests

Die Bestimmung des zytotoxischen Potentials der GB-Fusionsproteine wurde über die Substratumsetzung von gelbem Tetrazoliumsalz zu wasserlöslichem Formazanfarbstoff Zellen bestimmt (Barth, S. *et al.* 2000). Die relative Viabilität der Zellen wurde dabei über Positivkontrollen von mit Zeocin versetzten Zellen ermittelt.

In 96-Loch Flachbodenplatten wurden in Zwei-Vierfachansätzen jeweils Verdünnungsreihen des Toxins oder von Zellkulturüberständen angesetzt. Dazu wurden in jedes Loch der ersten Reihe 120 µl Überstand vorgelegt; In die restlichen Löcher der Verdünnungsreihe 100 µl Komplexmedium. Jeweils 20 µl von der ersten Reihe in die nächste Verdünnungsstufe pipettieren (1:5-Verdünnung). Positivkontrolle: 120 µl Komplexmedium mit Zeocin (100- 200 µg/ml); Negativkontrolle nur Komplexmedium. 1-0,8x10⁴ Zellen in 100 µl Komplexmedium wurden dann zu den Verdünnungsreihen pipettiert und 24-48 h bei 37°C in einem Brutschrank bei 5% CO₂ inkubiert. Nach Zugabe von 50 µl XTT/PMS (finale Konzentration von 0,3 mg und 0,383 ng) pro Loch und einer weiteren 4-48 h Inkubation der Zellen erfolgte die photometrische Messung der XTT-Substratumsetzung als Subtraktion von OD₄₅₀ₙₘ-OD₆₅₀ₙₘ in einem ELISA-Reader.

### Ergebnisse

### Granzym B-PCR

Neben den GB-spezifischen Sequenzen (Großbuchstaben) wurden über die Primer Restriktionsschnittstellen für die weitere Klonierung an das Amplifikationsprodukt angefügt (xx-Gb-back: *XbaI,* **XhoI;** Gb-for: **Cel II,** *BamHI)*
xx-Gb-back: gca*ctcgag***tctaga**ATCATCGGGGGACATGAGGCCAAG
**Gb-for:** ttcgt**gctcagc**tagttt*ggatcc***GTAGCGTTTCATGGTTTTCTTTATC**

Das Produkt der PCR zeigte die erwartete Länge von 720 bp (Fig. 2).

### Plasmidkonstruktionen

Alle Klonierungen des GB wurden über Xho I und Cel II in die verschiedenen verfügbaren pMS-Plasmide durchgeführt. Die Verifizierung der Klonierungen erfolgte über spezifische Restriktionsanalysen, der Sequenzierung von KGbII und dem immunhistochemischen Nachweis von KGbMH im Überstand transfizierter 293T Zellen (eg. pMS-KGbMH und pMS-KGb II) (Fig. 3).

### Sequenzierung

Eine erste Sequenzierung wurde anhand des GB-PCR-Produkts mit den GB-spezifischen Primern durchgeführt und bestätigte die GB-Sequenz. Die vollständige GB-Sequenz wurde auf Grundlage des pMS-KGb II-Plasmides ermittelt. Für die überlappende Sequenzierung in beide Richtungen wurden jeweils die GB-spezifische Primer (eg xx-Gb-back; Gb-for) sowie jeweils ein plasmidständiger Primer, ca. 100 bp 5' bzw. 3' der für die Klonierung relevanten Schnittstellen verwendet. Diese Sequenzierung zeigte eine 100%ige Homologie zu der in der Genbank von PubMed veröffentlichten GB-Sequenz unter der Accession-Nummer NM_004131.

### Expression der rekombinanten Proteine

Die Expression der Apoptotika erfolgte ausschließlich in eukaryontischen Zellen (eg. 293T). Fig. 4 zeigt das Ergebnis eines Western-Blot nach einem ProteinMiniprep.

### FACS-Analysen

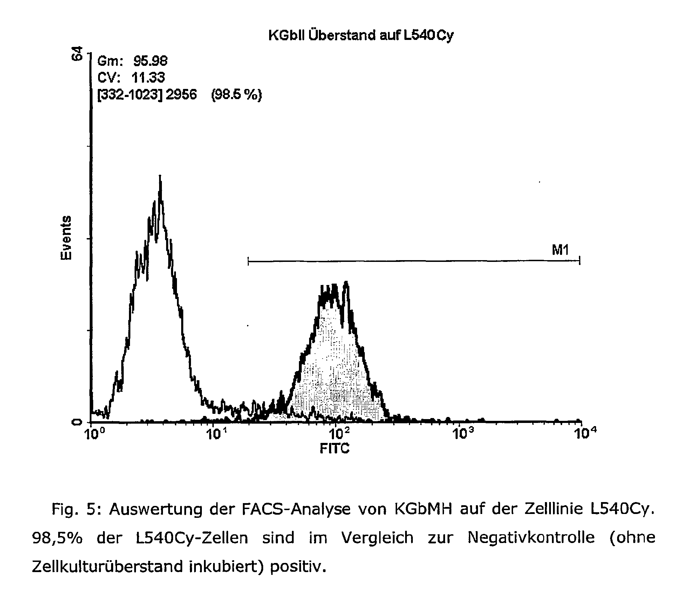

Um die Bindungsfähigkeit der eukaryontisch exprimierten KGB-Apoptotika zu evaluieren wurden FACS-Analysen auf CD30(+)- (eg L540Cy) und CD30(-)-Zelllinien (eg IMR5) durchgeführt. Auf der Negativzelllinie und den entsprechenden Kontrollen konnte keine Färbung der Zellen im FACS dokumentiert werden. Die Färbung der CD30(+)-Zellen mit den KGB-Apoptotika hingegen war identisch zu der Positivkontrolle mit Ki4moAk (Fig. 5). Dies zeigt die Ki-4(scFv) vermittelte Bindung der KGbMH-Apoptotika und das Fehlen unspezifischer Bindungen an die untersuchten Zellen.

### XTT-Viabilitätstests

### Kompetition von KGbMH mit Ki4-moAk

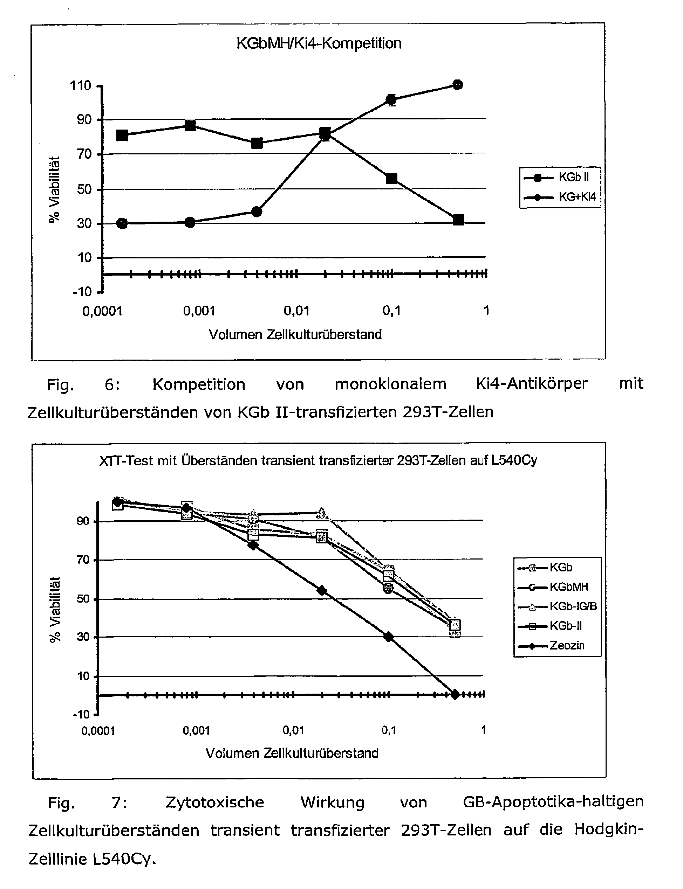

Neben den FACS-Analysen wurde eine Kompetition des KGbMH mit dem monoklonalem Ki-4-Antikörper auf L540Cy durchgeführt. Neben einem einfachen XTT-Viabilitätstest mit Zellkulturüberständen (KGb II) von stabil transfizierten 293T-Zellen, wurde eine Verdünnungsreihe von Ki4-moAk (Ausgangskonzentration 40 µg/ml) in 100 µl KGbMH-haltigen Zellkulturüberstand vorgelegt (KG+Ki4). Der spiegelsymmetrische Verlauf der Viabilitätskurven in Fig. 6 zeigt, dass KGbMH erfolgreich durch Ki-4-moAK blockiert werden kann.

### Überstände transient transfizierter 293T-Zellen

Der XTT-Viabilitätstest mit den Zellkulturüberständen von transient transfizierten 293T-Zellen diente der Klärung verschiedener zentraler Fragen:
- Sind humane/eukaryontische Zellen in der Lage, ein für sie potentiell toxisches Apoptotika zu produzieren und zu sezernieren?
- Ist die GB-Apoptotika-Syntheseleistung der transfizierten Zellen ausreichend, um einen zytotoxischen Effekt gegenüber den Zielzellen zu entfalten und ist damit ein potenzieller gentherapeutischer Einsatz ermöglicht?
- Inwieweit beeinflusst das C-terminale Anfügen von Markerepitopen (eg. Myc und His-Tag) die Funktionalität der GB-Apoptotika?
- Wird die GB-Apoptotika-Synthese durch die gleichzeitige Synthese des EGFP beeinträchtigt?

Je 1x10⁵ Zellen 293T wurden mit jeweils 1 µg Plasmid-DNA und 3 µl Transfast in einer 12-Loch Platte mit TransFast transfiziert. Es wurden jeweils 2 unabhängige Transfektionen parallel angesetzt. 72 h nach Transfektion wurden die Zellkulturüberstände mit jeweils 120 µl in der ersten Verdünnungsstufe eines XTT-Viabilitätstest eingesetzt (4 Reihen/Konstrukt). Die Messung erfolgte 48 h nach Zugabe von XTT/Phenacin. Die Auswertung des Viabilitätstest ist in Fig. 7 dargestellt.

Die dargestellten Ergebnisse zeigen, dass weder die vorgenommenen C-terminalen Modifikationen noch die gleichzeitige Expression des EGFP-Reportergens einen erkennbaren Einfluss auf die Funktionalität oder Quantität der sezernierten GB-Apoptotika haben.

Die Ergebnisse lassen zudem erwarten, dass GB-Apoptotika-kodierende und sezernierende Plasmide auch im Rahmen einer Gentherapie potenziell Verwendung finden können.

Neben den XTT-Viabilitätstests auf L540Cy wurden auch Kontrollen mit der CD30-negativen Zelllinie IMR5 durchgeführt. Hier konnte keine zytotoxische Wirkung der KGbMH-Apoptotika auf die Zellen beobachtet werden.

### Bestimmung der IC₅₀ von KGbMH

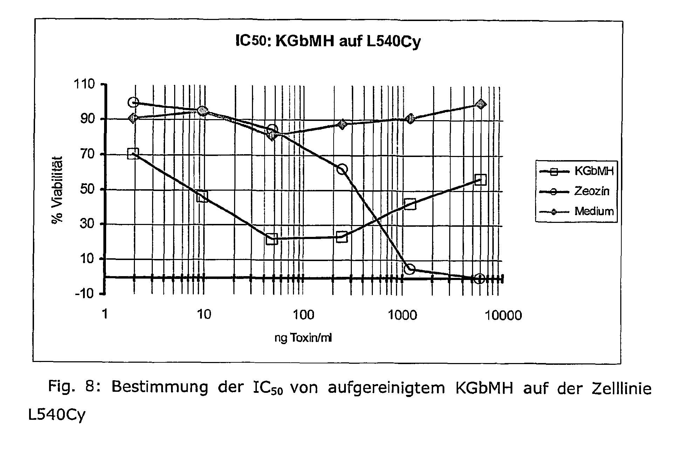

Nach Aufreinigung von KGbMH aus Zellkulturüberständen von pMS-KGb IItransfizierten Zellen wurde nach FACS-Analyse, Proteinmengenbestimmung sowie einer Reinheitsabschätzung der Präparation mit Hilfe eines Coomassie-Geles ein XTT-Viabilitätstest mit L540Cy angesetzt. Die anhand der grafischen Auswertung der Daten in Fig. 8 bestimmte IC₅₀ liegt für das KGbMH bei 7,5 ng/ml. Diese Größenordnung entspricht in etwa der von klassischen. Immuntoxinen, wie z.B. Ki-4(scvFv)-ETA'(3-6 ng/ml) (Barth, S. *et al.* 2000).

### Referenzen

Andrade, F., Roy, S., Nicholson, D., Thornberry, N., Rosen, A. and Casciola-Rosen, L. Granzyme B directly and efficiently cleaves several downstream caspase substrates: implications for CTL-induced apoptosis. Immunity8(4): 451-60 (1998).
Baluna, R. and Vitetta, E. S. Vascular leak syndrome: a side effect of immunotherapy. Immunopharmacology 37(2-3): 117-132 (1997).
Barth, S., Huhn, M., Matthey, B., Klimka, A., Galinski, E. A. and Engert, A. Compatible-solute-supported periplasmic expression of functional recombinant proteins under stress conditions. Appl Environ Microbiol 66(4): 1572-1579 (2000).
Barth, S., Huhn, M., Matthey, B., Klimka, A., Tawadros, S., Schnell, R., Diehl, V. and Engert, A. Ki-4(scFv)-ETA', a new recombinant anti-CD30 immunotoxin with highly specific cytotoxic activity against disseminated Hodgkin tumors in SCID mice. Blood 95: 3909-3914 (2000).
Barth, S., Huhn, M., Matthey, B., Tawadros, S., Schnell, R., Schinkothe, T., Diehl, V. and Engert, A. Ki-4(scFv)-ETA', a new recombinant anti-CD30 immunotoxin with highly specific cytotoxic activity against disseminated Hodgkin tumors in SCID mice. Blood 95(12): 3909-14 (2000).
Barth, S., Huhn, M., Wels, W., Diehl, V. and Engert, A. Construction and in vitro evaluation of RFT5(scFv)-ETA', a new recombinant single-chain immunotoxin with specific cytotoxicity toward CD25+ Hodgkin-derived cell lines. Int J Mol Med 1(1): 249-256 (1998).
Battelli, M. G., Buonamici, L., Polito, L., Bolognesi, A. and Stirpe, F. Hepatoxicity of ricin, saporin or a saporin immunotoxin: xanthine oxidase activity in rat liver and blood serum. Virchows Arch 427(5): 529-35 (1996).
Beaumelle, B., Bensammar, L. and Bienvenue, A. Selective translocation of the A chain of diphtheria toxin across the membrane of purified endosomes. J Biol Chem 267(16): 11525-11531 (1992).
Beresford, P. J., Xia, Z., Greenberg, A. H. and Lieberman, J. Granzyme A loading induces rapid cytolysis and a novel form of DNA damage independently of caspase activation [published erratum appears in Immunity 1999 Jun;10(6):following 768]. Immunity 10(5): 585-94 (1999).
Bolognesi, A., Tazzari, P. L., Olivieri, F., Polito, L., Falini, B. and Stirpe, F. Induction of apoptosis by ribosome-inactivating proteins and related immunotoxins. Int J Cancer 68(3): 349-55 (1996).
Brenner, T., Steinberger, I., Soffer, D., Beraud, E., Ben-Nun, A. and Lorberboum-Galski, H. A novel antigen-toxin chimeric protein: myelin basic protein- pseudomonas exotoxin (MBP-PE 40) for treatment of experimental autoimmune encephalomyelitis. Immunol Lett 68(2-3): 403-10 (1999).
Bukovsky, J., Evans, A., Tartaglione, M. and Kennett, R. H. Selection of variant neuroblastoma cell line which has lost cell surface expression of antigen detected by monoclonal antibody PI153/3. Somat Cell Mol Genet 11(5): 517-22 (1985).
Chaudhary, V., Batra, J., Gallo, M., Willingham, M., Fitz, G. D. and Pastan, I. A rapid method of cloning functional variable-region antibody genes in Escherichia coli as single-chain immunotoxins [published erratum appears in Proc Natl Acad Sci U S A 1990 Apr; 87(8):3253]. Proc Natl Acad Sci USA 87(3): p1066-70 (1990).
Chaudhary, V., Gallo, M., Fitz, G. D. and Pastan, I. A recombinant single-chain immunotoxin composed of anti-Tac variable regions and a truncated diphtheria toxin. Proc Natl Acad Sci U S A 87(23): p9491-4 (1990).
Chaudhary, V., Queen, C., Junghans, R., Waldmann, T., Fitz, G. D. and Pastan, I. A recombinant immunotoxin consisting of two antibody variable domains fused to Pseudomonas exotoxin. Nature 339(6223): p394-7 (1989).
Chen, S., Yang, A., Chen, J., Kute, T., King, C., Collier, J., Cong, Y., Yao, C. and Huang, X. Potent antitumour activity of a new class of tumour-specific killer cells. Nature 385(6611): p78-80 (1997).
Crowe, J., Dobeli, H., Gentz, R., Hochuli, E., Stuber, D. and Henco, K. 6xHis-Ni-NTA chromatography as a superior technique in recombinant protein expression/purification. Methods Mol Biol 31: 371-87 (1994).
Endo, Y., Mitsui, K., Motizuki, M. and Tsurugi, K. The mechanism of action of ricin and related toxic lectins on eukaryotic ribosomes. The site and the characteristics of the modification in 28 S ribosomal RNA caused by the toxins. J Biol Chem 262(12): 5908-12 (1987).
Engert, A., Diehl, V., Schnell, R., Radszuhn, A., Hatwig, M. T., Drillich, S., Schon, G., Bohlen, H., Tesch, H., Hansmann, M. L., Barth, S., Schindler, J., Ghetie, V., Uhr, J. and Vitetta, E. A phase-I study of an anti-CD25 ricin A-chain immunotoxin (RFT5-SMPT-dgA) in patients with refractory Hodgkin's lymphoma. Blood 89(2): 403-410 (1997).
Fernandes-Alnemri, T., Armstrong, R. C., Krebs, J., Srinivasula, S. M., Wang, L., Bullrich, F., Fritz, L. C., Trapani, J. A., Tomaselli, K. J., Litwack, G. and Alnemri, E. S. In vitro activation of CPP32 and Mch3 by Mch4, a novel human apoptotic cysteine protease containing two FADD-like domains. Proc Natl Acad Sci U S A 93(15): 7464-9 (1996).
Fett, J. W., Strydom, D. J., Lobb, R. R., Alderman, E. M., Bethune, J. L., Riordan, J. F. and Vallee, B. L. Isolation and characterization of angiogenin, an angiogenic protein from human carcinoma cells. Biochemistry 24(20): 5480-6 (1985).
FitzGerald, D. J., Willingham, M. C. and Pastan, I. Pseudomonas exotoxinimmunotoxins. Cancer Treat Res 37: 161-173 (1988).
Graham, F. L., Smiley, J., Russell, W. C. and Nairn, R. Characteristics of a human cell line transformed by DNA from human adenovirus type 5. J Gen Virol 36(1): 59-74 (1977).
Greenberg, A. H. Granzyme B-induced apoptosis. Adv Exp Med Biol 406: 219-28 (1996).
Grossbard, M. L., Fidias, P., Kinsella, J., O'Toole, J., Lambert, J. M., Blattler, W. A., Esseltine, D., Braman, G., Nadler, L. M. and Anderson, K. C. Anti-B4-blocked ricin: a phase II trial of 7 day continuous infusion in patients with multiple myeloma. BrJ Haematol 102(2): 509-515 (1998).
Grossbard, M. L., Lambert, J. M., Goldmacher, V. S., Spector, N. L., Kinsella, J., Eliseo, L., Coral, F., Taylor, J. A., Blattler, W. A. and Epstein, C. L. Anti-B4-blocked ricin: a phase I trial of 7-day continuous infusion in patients with B-cell neoplasms. J Clin Oncol 11(4): 726-737 (1993).
Harkonen, S., Stoudemire, J., Mischak, R., Spitler, L. E., Lopez, H. and Scannon, P. Toxicity and immunogenicity of monoclonal antimelanoma antibody-ricin A chain immunotoxin in rats. Cancer Res 47(5): 1377-82 (1987).
Hochuli, V. Cholesterol testing for all [letter]. Bmj 298(6677): 891 (1989).
Iglewski, B. H. and Kabat, D. NAD-dependent inhibition of protein synthesis by Pseudomonas aeruginosa toxin. Proc Natl Acad Sci U S A 72(6): 2284-8 (1975).
Jans, D. A., Jans, P., Briggs, L. J., Sutton, V. and Trapani, J. A. Nuclear transport of granzyme B (fragmentin-2). Dependence of perforin in vivo and cytosolic factors in vitro. J Biol Chem 271(48): 30781-9 (1996).
Jurcic, J. G. and Scheinberg, D. A. Radioimmunotherapy of hematological cancer: problems and progress. Clin Cancer Res 1(12): 1439-1446 (1995).
Kam, C. M., Hudig, D. and Powers, J. C. Granzymes (lymphocyte serine proteases): characterization with natural and synthetic substrates and inhibitors. Biochim Biophys Acta 1477(1-2): 307-23 (2000).
Kaminski, M. S., Zasadny, K. R., Francis, I. R., Fenner, M. C., Ross, C. W., Milik, A. W., Estes, J., Tuck, M., Regan, D., Fisher, S., Glenn, S. D. and Wahl, R. L. Iodine-131-anti-B1 radioimmunotherapy for B-cell lymphoma. J Clin Oncol 14(7): 1974-1981 (1996).
Kapp, U., Wolf, J., von Kalle, C., Tawadros, S., Rottgen, A., Engert, A., Fonatsch, C., Stein, H. and Diehl, V. Preliminary report: growth of Hodgkin's lymphoma derived cells in immune compromised mice. Ann Oncol 3 Suppl 4 : 21-23 (1992).
Keppler-Hafkemeyer, A., Brinkmann, U. and Pastan, I. Role of caspases in immunotoxin-induced apoptosis of cancer cells. Biochemistry 37(48): 16934-16942 (1998).
Keppler-Hafkemeyer, A., Kreitman, R. J. and Pastan, I. Apoptosis induced by immunotoxins used in the treatment of hematologic malignancies. Int J Cancer 87(1): 86-94 (2000).
Khazaeli, M. B., Conry, R. M. and Lo Buglio, A. F. Human immune response to monoclonal antibodies. J Immunother 15(1): 42-52 (1994).
Klimka, A., Barth, S., Drillich, S., Wels, W., van Snick, J., Renauld, J. C., Tesch, H., Bohlen, H., Diehl, V. and Engert, A. A deletion mutant of Pseudomonas exotoxin-A fused to recombinant human interleukin-9 (rhIL-9-ETA') shows specific cytotoxicity against IL-9-receptor-expressing cell lines. Cytokines Mol Ther 2(3): 139-146 (1996).
Kreitman, R. J., Wilson, W. H., White, J. D., Stetler-Stevenson, M., Jaffe, E. S., Giardina, S., Waldmann, T. A. and Pastan, I. Phase I trial of recombinant immunotoxin anti-Tac(Fv)-PE38 (LMB-2) in patients with hematologic malignancies. J Clin Oncol 18(8): 1622-1636 (2000).
Kuzel, T. M., Rosen, S. T., Gordon, L. I., Winter, J., Samuelson, E., Kaul, K., Roenigk, H. H., Nylen, P. and Woodworth, T. Phase I trial of the diphtheria toxin/interleukin-2 fusion protein DAB486IL-2: efficacy in mycosis fungoides and other non-Hodgkin's lymphomas. Leuk Lymphoma 11(5-6): 369-377 (1993).
Leland, P. A., Schultz, L. W., Kim, B. M. and Raines, R. T. Ribonuclease A variants with potent cytotoxic activity. Proc Natl Acad Sci U S A 95(18): 10407-12 (1998).
LeMaistre, C., Saleh, M., Kuzel, T., Foss, F., Platanias, L., Schwartz, G., Ratain, M., Rook, A., Freytes, C., Craig, F., Reuben, J. and Nichols, J. Phase I trial of a ligand fusion-protein (DAB389IL-2) in lymphomas expressing the receptor for interleukin-2. Blood 91(2): p399-405 (1998).
LeMaistre, C. F., Meneghetti, C., Rosenblum, M., Reuben, J., Parker, K., Shaw, J., Deisseroth, A., Woodworth, T. and Parkinson, D. R. Phase I trial of an interleukin-2 (IL-2) fusion toxin (DAB486IL-2) in hematologic malignancies expressing the IL-2 receptor. Blood 79(10): 2547-2554 (1992).
LoBuglio, A. F. and Saleh, M. N. Monoclonal antibody therapy of cancer. Crit Rev Oncol Hematol 13(3): 271-82 (1992).
Lowry, O. H., Rosebrough, N. J., Farr, A. L. and Randall, R. J. Protein Measurement with the Folin Phenol Reagent. Journal of Biological Chemistry 193: 265-275 (1951).
Matthey, B., Engert, A. and Barth, S. Ki-4(scFv)-Ricin, a new recombinant immunotoxin targeting CD30 isolated from E.coli periplasmic space exhibits specific cytotoxic activity against Hodgkin cells in vitro. submitted (2000).
Newton, D., Nicholls, P., Rybak, S. and Youle, R. Expression and characterization of recombinant human eosinophil-derived neurotoxin and eosinophil-derived neurotoxin-anti-transferrin receptor sFv. J Biol Chem 269(43): p26739-45 (1994).
Newton, D., Xue, Y., Olson, K., Fett, J. and Rybak, S. Angiogenin single-chain immunofusions: influence of peptide linkers and spacers between fusion protein domains. Biochemistry 35(2): p545-53 (1996).
Newton, D. L., Ilercil, O., Laske, D. W., Oldfield, E., Rybak, S. M. and Youle, R. J. Cytotoxic ribonuclease chimeras. Targeted tumoricidal activity in vitro and in vivo. J Biol Chem 267(27): 19572-8 (1992).
Nicholson, D. W. and Thornberry, N. A. Caspases: killer proteases. Trends Biochem Sci 22(8): 299-306 (1997).
O'Hare, M., Brown, A. N., Hussain, K., Gebhardt, A., Watson, G., Roberts, L. M., Vitetta, E. S., Thorpe, P. E. and Lord, J. M. Cytotoxicity of a recombinant ricin-A-chain fusion protein containing a proteolytically-cleavable spacer sequence. FEBS Lett 273(1-2): 200-4 (1990).
Pai, L. H. and Pastan, I. Clinical trials with Pseudomonas exotoxin immunotoxins. Curr Top Microbiol Immunol 234: 83-96 (1998).
Pinkoski, M. J., Heibein, J. A., Barry, M. and Bleackley, R. C. Nuclear translocation of granzyme B in target cell apoptosis. Cell Death Differ 7(1): 17-24 (2000).
Pirker, R. Immunotoxins against solid tumors. J Cancer Res Clin Oncol. 114(4): 385-93 (1988).
Porath, J., Carlsson, J., Olsson, I. and Belfrage, G. Metal chelate affinity chromatography, a new approach to protein fractionation. Nature 258(5536): 598-9 (1975).
Press, O. W., Eary, J., Badger, C. C., Appelbaum, F. R., Wiseman, G., Matthews, D., Martin, P. J. and Bernstein, I. D. High-dose radioimmunotherapy of lymphomas. Cancer Treat Res 68: 13-22 (1993).
Rybak, S., Hoogenboom, H., Meade, H., Raus, J., Schwartz, D. and Youle, R. Humanization of immunotoxins. Proc Natl Acad Sci U S A 89(8): p3165-9 (1992).
Rybak, S., Saxena, S., Ackerman, E. and Youle, R. Cytotoxic potential of ribonuclease and ribonuclease hybrid proteins. J Biol Chem 266(31): p21202-7 (1991).
Saleh, M. N., Khazaeli, M. B., Wheeler, R. H., Dropcho, E., Liu, T., Urist, M., Miller, D. M., Lawson, S., Dixon, P., Russell, C. H. and et al. Phase I trial of the murine monoclonal anti-GD2 antibody 14G2a in metastatic melanoma. Cancer Res 52(16): 4342-7 (1992).
Sambrook, J., Fritsch, E. and Maniatis, T. (1989). Molecular cloning. A laboratory manual. New York, Cold Spring Harbor Laboratory Press.
Sanger, F., Nicklen, S. and Coulson, A. R. DNA sequencing with chainterminating inhibitors. Proc Natl Acad Sci USA 74(12): 5463-5467 (1977).
Sarin, A., Williams, M. S., Alexander-Miller, M. A., Berzofsky, J. A., Zacharchuk, C. M. and Henkart, P. A. Target cell lysis by CTL granule exocytosis is independent of ICE/Ced-3 family proteases. Immunity 6(2): 209-15 (1997).
Saxena, S. K., Rybak, S. M., Davey, R. T. J., Youle, R. J. and Ackerman, E. J. Angiogenin is a cytotoxic, tRNA-specific ribonuclease in the RNase A superfamily. J Biol Chem 267(30): 21982-21986 (1992).
Schnell, R., Vitetta, E., Schindler, J., Barth, S., Winkler, U., Borchmann, P., Hansmann, M. L., Diehl, V., Ghetie, V. and Engert, A. Clinical trials with an anti-CD25 ricin A-chain experimental and immunotoxin (RFT5-SMPT-dgA) in Hodgkin's lymphoma. Leuk Lymphoma 30(5-6): 525-537 (1998).
Schnell, R., Vitetta, E., Schindler, J., Borchmann, P., Barth, S., Ghetie, V., Hell, K., Drillich, S., Diehl, V. and Engert, A. Treatment of refractory Hodgkin's lymphoma patients with an anti-CD25 ricin A-chain immunotoxin. Leukemia 14(1): 129-135 (2000).
Shapiro, R., Riordan, J. F. and Vallee, B. L. The characteristic ribonuclease activity of angiogenin. Biochem. Insert 25: 3527-3532 (1986).
Shresta, S., Pham, C. T., Thomas, D. A., Graubert, T. A. and Ley, T. J. How do cytotoxic lymphocytes kill their targets? Curr Opin Immunol 10(5): 581-7 (1998).
Srinivasula, S. M., Fernandes-Alnemri, T., Zangrilli, J., Robertson, N., Armstrong, R. C., Wang, L., Trapani, J. A., Tomaselli, K. J., Litwack, G. and Alnemri, E. S. The Ced-3/interleukin 1beta converting enzyme-like homolog Mch6 and the lamin-cleaving enzyme Mch2alpha are substrates for the apoptotic mediator CPP32. J Biol Chem 271(43): 27099-106 (1996).
Sutton, V. R., Davis, J. E., Cancilla, M., Johnstone, R. W., Ruefli, A. A., Sedelies, K., Browne, K. A. and Trapani, J. A. Initiation of apoptosis by granzyme B requires direct cleavage of bid, but not direct granzyme B-mediated caspase activation [In Process Citation]. J Exp Med 192(10): 1403-14 (2000).
Thorpe, P. E., Wallace, P. M., Knowles, P. P., Relf, M. G., Brown, A. N., Watson, G. J., Blakey, D. C. and Newell, D. R. Improved antitumor effects of immunotoxins prepared with deglycosylated ricin A-chain and hindered disulfide linkages. Cancer Res 48(22): 6396-6403 (1988).
Trapani, J. A., Jans, D. A., Jans, P. J., Smyth, M. J., Browne, K. A. and Sutton, V. R. Efficient nuclear targeting of granzyme B and the nuclear consequences of apoptosis induced by granzyme B and perforin are caspase-dependent, but cell death is caspäse-independent. J Biol Chem 273(43): 27934-8 (1998).
Trapani, J. A., Jans, P., Smyth, M. J., Froelich, C. J., Williams, E. A., Sutton, V. R. and Jans, D. A. Perforin-dependent nuclear entry of granzyme B precedes apoptosis, and is not a consequence of nuclear membrane dysfunction. Cell Death Differ 5(6): 488-96 (1998).
Vitetta, E. S. Immunotoxins and vascular leak syndrome [In Process Citation]. Cancer J Sci Am 6 Suppl 3: S218-224 (2000).
Yokota, T., Milenic, D. E., Whitlow, M. and Schlom, J. Rapid tumor penetration of a single-chain Fv and comparison with other immunoglobulin forms. Cancer Res 52(12): 3402-3408 (1992).
Yoon, J. M., Han, S. H., Kown, O. B., Kim, S. H., Park, M. H. and Kim, B. K. Cloning and cytotoxicity of fusion proteins of EGF and angiogenin. Life Sci 64(16): 1435-1445 (1999).
Youle, R. J. and Neville, D. M. J. Anti-Thy 1.2 monoclonal antibody linked to ricin is a potent cell-type-specific toxin. Proc Natl Acad Sci USA 77(9): 5483-5486 (1980).
Zewe, M., Rybak, S., Dubel, S., Coy, J., Welschof, M., Newton, D. and Little, M. Cloning and cytotoxicity of a human pancreatic RNase immunofusion. Immunotechnology 3(2): p127-36 (1997).

## Patentansprüche

1. Rekombinantes Fusionsprotein gebildet aus mindestens einer Komponente A und mindestens einer Komponente B, **dadurch gekennzeichnet, dass** die Komponente A eine Bindungsaktivität für zelluläre Oberflächenstrukturen und Komponente B ein Granzym ist.

2. Fusionsprotein nach Anspruch 1, die neben den Komponenten A und B eine oder mehrere supplementäre Komponenten S enthalten.

3. Fusionsprotein nach Ansprüchen 1 und/oder 2, **dadurch gekennzeichnet, dass** die Komponente A ausgewählt wird aus der Gruppe aktiv bindender Strukturen, bestehend aus Antikörpern oder deren Derivate oder Fragmente, synthetische Peptide oder chemische Moleküle, Liganden, Lektine, Rezeptorbindungsmoleküle, Cytokine, Lymphokine, Chemokine, Adhäsionsmoleküle und deren Derivate, Mutanten oder Kombinationen davon, die an Cluster of Differentiation Antigene, Zytokin-, Hormon-, Wachstumsfaktor-Rezeptoren, Ionenpumpen, kanalbildende Proteine binden.

4. Fusionsprotein nach Ansprüchen 1 und 2, **dadurch gekennzeichnet, dass** die Komponente A ausgewählt wird aus der Gruppe passiv gebundener Strukturen bestehend aus Allergenen, peptidischen Allergenen, rekombinanten Allergenen, allergenidiotypischen Antikörpern, Strukturen, die eine Autoimmunreaktion provozieren oder eine Gewebeabstoßungsreaktion induzieren und deren Derivate, Mutanten oder Kombinationen davon.

5. Fusionsprotein nach Ansprüchen 1 bis 4, **dadurch gekennzeichnet, dass** die Komponente B proteolytische Eigenschaften besitzt oder mindestens eine Protease, deren Derivate, Mutante oder Kombination ist.

6. Fusionsprotein nach Ansprüchen 1 bis 5, **dadurch gekennzeichnet, dass** die Komponente B Komponenten der zellinhärenten Apoptose direkt aktiviert und damit die Apoptose in den über die Bindung von Komponente A definierten Zellen induziert.

7. Fusionsprotein nach Ansprüchen 1 bis 6, **dadurch gekennzeichnet, dass** die Komponente B das Granzym B oder ein Derivat davon ist.

8. Fusionsprotein nach Ansprüchen 1 bis 7, **dadurch gekennzeichnet, dass** eine Komponente S die Syntheseleistung reguliert.

9. Fusionsprotein nach Anspruch 8, **dadurch gekennzeichnet, dass** die Komponente S die Aufreinigung des Komplexes oder seiner Komponenten gewährleistet.

10. Fusionsprotein nach Ansprüchen 7 bis 9, **dadurch gekennzeichnet, dass** die Komponente S die Translokation in Zielzellen unterstützt.

11. Fusionsprotein nach Ansprüchen 7 bis 10, **dadurch gekennzeichnet, dass** die Komponente S eine intrazelluläre Aktivierung der Komponente B ermöglicht.

12. Nukleinsäuremoleküle oder Vektoren, die für ein Fusionsprotein nach Anspruch 11 kodieren.

13. Organismen, ausgenommen der Mensch, **dadurch gekennzeichnet, dass** mit ihnen eine Transformation oder Transfektion mit Nukleinsäuremolekülen oder Vektoren nach Anspruch 12 erfolgte und dass sie Fusionsproteine gemäß Anspruch 1 synthetisieren.

14. Arzneimittel enthaltend ein Fusionsprotein nach einem der Ansprüche 1 bis 11 oder Nukleinsäuremoleküle nach Anspruch 12.

15. Verwendung des Fusionsproteins nach einem der Ansprüche 1 bis 11und/oder der dafür kodierenden Nukleinsäuremoleküle nach Anspruch 12 zur Herstellung eines Arzneimittels zur Behandlung maligner Erkrankungen, Allergien, Autoimmunreaktionen, chronischen Entzündungsreaktionen oder Gewebeabstoßungsreaktionen.

## Claims

1. A recombinant fusion protein formed from at least one component A and at least one component B, **characterized in that** component A has a binding activity for cellular surface structures, and component B is a granzyme.

2. The fusion protein according to claim 1, containing one or more supplementary components S in addition to said components A and B.

3. The fusion protein according to claims 1 and/or 2, **characterized in that** component A is selected from the group of actively binding structures consisting of antibodies or their derivatives or fragments, synthetic peptides or chemical molecules, ligands, lectins, receptor binding molecules, cytokines, lymphokines, chemokines, adhesion molecules and their derivatives, mutants or combinations thereof, which bind to cluster of differentiation antigens, cytokine, hormone, growth factor receptors, ion pumps, channel-forming proteins.

4. The fusion protein according to claims 1 and 2, **characterized in that** component A is selected from the group of passively bound structures consisting of allergens, peptidic allergens, recombinant allergens, allergen-idiotypical antibodies, autoimmune-provoking structures, tissue-rejection-inducing structures and their derivatives, mutants or combinations thereof.

5. The fusion protein according to claims 1 to 4, **characterized in that** component B has proteolytic properties or is at least one protease, their derivatives, mutant or combination.

6. The fusion protein according to claims 1 to 5, **characterized in that** component B directly activates components of cell-inherent apoptosis and thus induces apoptosis in the cells defined through the binding of component A.

7. The fusion protein according to claims 1 to 6, **characterized in that** component B is granzyme B or a derivative thereof.

8. The fusion protein according to claims 1 to 7, **characterized in that** a component S regulates the synthetic performance.

9. The fusion protein according to claim 8, **characterized in that** said component S enables the purification of the fusion protein or its components.

10. The fusion protein according to claims 7 to 9, **characterized in that** said component S supports the translocation into target cells.

11. The fusion protein according to claims 7 to 10, **characterized in that** said component S enables intracellular activation of component B.

12. Nucleic acid molecules or vectors coding for a fusion protein according to claim 11.

13. Organisms, except for humans, **characterized by** having been transformed or transfected with nucleic acid molecules or vectors according to claim 12 and by synthesizing fusion proteins according to claim 1.

14. A medicament containing a fusion protein according to any of claims 1 to 11 or nucleic acid molecules according to claim 12.

15. Use of the fusion protein according to any of claims 1 to 11 and/or the nucleic acid molecules coding therefor according to claim 12 for preparing a medicament for treating malignant diseases, allergies, autoimmune reactions, chronic inflammation reactions or tissue rejection reactions.

## Revendications

1. Protéine de fusion recombinante formée d'au moins un composant A et d'au moins un composant B, **caractérisée en ce que** le composant A a une activité de liaison pour les structures de surfaces cellulaires et le composant B est un granzyme.

2. Protéine de fusion selon la revendication 1, qui comprend, en plus des composants A et B, un ou plusieurs composants S supplémentaires.

3. Protéine de fusion selon les revendications 1 et/ou 2, **caractérisée en ce que** le composant A est choisi dans le groupe constitué par les structures à liaison active, comprenant les anticorps ou leurs dérivés ou fragments, les peptides synthétiques ou les molécules chimiques, les ligands, les lectines, les molécules de liaison aux récepteurs, les cytokines, les lymphokines, les chimiokines, les molécules d'adhérence et leurs dérivés, les mutants ou les combinaisons de ceux-ci, qui se lient aux antigènes CD (Cluster of Differentiation), aux récepteurs des cytokines, des hormones ou des facteurs de croissance, aux pompes ioniques, aux protéines formant des canaux.

4. Protéine de fusion selon les revendications 1 et 2, **caractérisée en ce que** le composant A est choisi dans le groupe constitué par les structures à liaison passive, comprenant les allergènes, les allergènes peptidiques, les allergènes recombinés, les anticorps anti-idiotypiques des allergènes, les structures qui induisent une réaction autoimmune ou une réaction de rejet de tissus, et leurs dérivés, leurs mutants ou des combinaisons de ceux-ci.

5. Protéine de fusion selon les revendications 1 à 4, **caractérisée en ce que** le composant B possède des propriétés protéolytiques ou est au moins une protéase, un de ses dérivés, mutants ou combinaison.

6. Protéine de fusion selon les revendications 1 à 5, **caractérisée en ce que** le composant B active directement des composants de l'apoptose cellulaire et induit donc l'apoptose dans les cellules définies par la liaison du composant A.

7. Protéine de fusion selon les revendications 1 à 6, **caractérisée en ce que** le composant B est le granzyme B ou un dérivé de celui-ci.

8. Protéine de fusion selon les revendications 1 à 7, **caractérisée en ce qu'**un composant S régule la capacité de synthèse.

9. Protéine de fusion selon la revendication 8, **caractérisée en ce que** le composant S garantit la purification du complexe ou de ses composants.

10. Protéine de fusion selon les revendications 7 à 9, **caractérisée en ce que** le composant S favorise la translocation dans les cellules cibles.

11. Protéine de fusion selon les revendications 7 à 10, **caractérisée en ce que** le composant S permet une activation intracellulaire du composant B.

12. Molécules d'acide nucléique ou vecteurs qui codent pour une protéine de fusion selon la revendication 11.

13. Organismes, à l'exception de l'être humain, **caractérisés en ce qu'**ils sont utilisés pour une transformation ou une transfection avec des molécules d'acide nucléique ou des vecteurs selon la revendication 12 et **en ce qu'**ils synthétisent des protéines de fusion selon la revendication 1.

14. Médicament contenant une protéine de fusion selon l'une quelconque des revendications 1 à 11 ou une molécule d'acide nucléique selon la revendication 12.

15. Utilisation de la protéine de fusion selon l'une quelconque des revendications 1 à 11 et/ou de la molécule d'acide nucléique codant pour celle-ci selon la revendication 12, pour la fabrication d'un médicament destiné au traitement de maladies malignes, d'allergies, de réactions autoimmunes, de réactions inflammatoires chroniques ou de réactions de rejet de tissus.


### SEQUENZPROTOKOLL

<110> Barth, Stefan
   Engert, Andreas
   Stöcker, Michael
<120> Apoptotika
<130> 010582wo
<140> PCT/EP/01/04514
   <141> 2001-04-20
<150> DE10020095.8
   <151> 2000-04-22
<160> 2
<170> PatentIn Ver. 2.1
<210> 1
   <211> 39
   <212> DNA
   <213> Künstliche Sequenz
<220>
   <223> Beschreibung der künstlichen Sequenz: Primer xx-Gb-back zur Klonierung
<400> 1
   gcactcgagt ctagaatcat cgggggacat gaggccaag 39
<210> 2
   <211> 49
   <212> DNA
   <213> Künstliche Sequenz
<220>
   <223> Beschreibung der künstlichen Sequenz: Primer Gb-for zur Klonierung
<400> 2
   ttcgtgctca gctagtttgg atccgtagcg tttcatggtt ttctttatc 49